# EUROPEAN PATENT APPLICATION

(11) **EP 4 400 151 A1**
(43) Date of publication of application: **17.07.2024**
(21) Application number: 23382015.8
(22) Date of filing: 11.01.2023
(51) Int. Cl.: A61M 25/06, A61M 29/00

(54) **CATHETER DEVICES, KITS AND METHODS**

(71) Applicant: Vascular Barcelona Devices, S.L., 08006 Barcelona (ES)
(72) Inventor: LLUSÀ MELÉNDEZ, Guiu, 08018 BARCELONA (ES); ROCHE REBOLLO, Enrique, 08034 BARCELONA (ES)
(74) Representative: Bardehle Pagenberg S.L.

(57) **Abstract**

The present disclosure relates to a catheter device configured to provide access to a hollow organ comprising a catheter body. The catheter body comprises a needle channel extending substantially along a longitudinal direction of the catheter body and comprising a distal opening at the distal end of the catheter body. The needle channel is configured to receive a removable elongated needle for making a puncture. The catheter body further comprises a dilator portion arranged around the needle channel and configured to enter into the puncture in the skin made by the removable elongated needle and configured to dilate the skin around the puncture. The distal opening of the needle channel is formed by a bevelled distal tip of the dilator portion, and the dilator portion further comprises a tapered outer surface extending from the bevelled distal tip in a proximal direction and a helix arranged around the tapered outer surface.

## Description

The present disclosure relates to catheter devices configured to provide access to hollow organs. The present disclosure further relates to kits including such catheter devices and methods for using catheter devices, in particular for accessing hollow organs.

### BACKGROUND

Medical procedures to obtain access through the patient's skin to hollow organs such as e.g. blood vessels are widely used and for many different kinds of interventions, e.g. endovascular repair, angioplasty, insertion of chest drains etc.

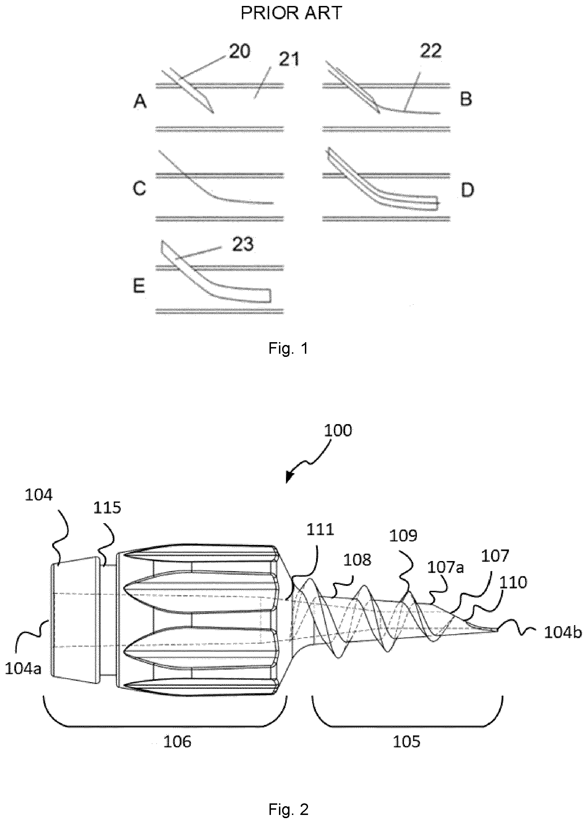

One known technique used in such procedures is the Seldinger technique which can be used e.g. for vascular access, or for placement of pleural, peritoneal, cardiac, and enteral drains and tubes. This procedure usually involves the following steps depicted in figure 1: a puncture is performed with an introducer needle (or "trocar") 20 placed through the patient's skin and into the hollow organ 21 of concern (step A). A soft-tipped guide wire 22 is then passed through the lumen of the introducer needle 20 and brought into a desired position within the hollow organ 21 (step B). Then, the needle 20 is withdrawn while the guide wire 22 remains in the hollow organ 21 (step C). At this point of the procedure, a sheath e.g. a blunt cannula or other devices which allows investigation or treatment of the hollow organ may be passed over the guide wire (step D). In examples, a dilator may be used before positioning a sheath. Once the sheath 23 is placed in the correct position for investigation or treatment of the hollow organ, the wire is removed (step E).

The sheaths by necessity are larger than the guide wire over which they are passed and hence cannot pass through the skin via the small hole made by the initial needle puncture, thus an enlargement of the puncture site may be needed. For this reason, a small skin incision is made adjacent to the puncture site i.e. the point of entry of the guide wire into the skin. This enlargement of the puncture site may be made with a sharp instrument e.g. a surgical scalpel. However, this requires great dexterity and it is difficult to make a precise incision. Accidents can occur if the incision is made too deep or too long in case the surgeon does not have a good enough control. This may lead to hemorrhage or perforation of the hollow organ (e.g. vein). In addition, creating and enlarging the puncture site may be time consuming, which increases stress for the patient. There is also the risk of cutting the guide wire, which is a very thin wire. And it also happens that a cut is made which is not exactly at the puncture site. Moreover the procedure supposes a risk for accidental injury for the practitioner.

The Seldinger technique may be used for angiography, insertion of chest drains and central venous catheters, insertion of PEG tubes using the push technique, insertion of the leads for an artificial pacemaker or implantable cardioverter-defibrillator, and numerous other interventional medical procedures.

WO2016015787 discloses a catheter device configured to provide access to a hollow organ. It includes a body extending longitudinally from a proximal end to a distal end and a flexible insertion tube extending longitudinally from a proximal end to a distal end, wherein the body comprises: an elongated channel along the longitudinal length of the body extending from the proximal end of the body to the distal end of the body, wherein the channel is configured to receive an elongate needle. The body further comprises one or more cutting edges extending rearwardly from a distal end of the body. Furthermore, the flexible insertion tube of the catheter device is coupled to the distal end of the body and comprises a tubular elongated passage aligned with the tubular elongated channel of the body forming a lumen.

WO2018024675 discloses a catheter device configured to provide access to a hollow organ. It comprises a catheter body extending longitudinally from a proximal end to a distal end, wherein the body comprises: an elongate channel extending substantially along a longitudinal direction of the catheter body from the proximal end to the distal end of the catheter body, wherein the channel is configured to receive an elongated needle of a removable needle device. The body further comprises one or more cutting edges extending in a proximal direction from a distal end of the catheter body. In addition, the body comprises one or more recesses adapted to mate with one or more catches of the needle assembly for coupling the needle assembly to the catheter device.

These previous catheter devices facilitate providing access to a hollow organ without the need of a surgical knife or scalpel.

It is an object of the present disclosure to provide catheter devices, (surgical) kits and methods that further improve the prior art and are easier to use by medical professionals.

### SUMMARY

In a first aspect, a catheter device configured to provide access to a hollow organ is provided. The catheter device comprises a catheter body extending longitudinally from a proximal end to a distal end. The catheter body comprises a needle channel arranged within the catheter body, extending substantially along a longitudinal direction of the catheter body and comprising a distal opening at the distal end of the catheter body. The needle channel is configured to receive a removable elongated needle for making a puncture in a skin when arranged in the needle channel. The catheter body further comprises a dilator portion arranged around the needle channel and configured to enter into a puncture in the skin made by the removable elongated needle. The dilator portion is configured to dilate the skin around the puncture. The distal opening of the channel is formed by a bevelled distal tip of the dilator portion. The dilator portion further comprises a tapered outer surface extending from the bevelled distal tip in a proximal direction and a helix arranged around the tapered outer surface.

According to this first aspect, a catheter device that is configured to provide a double function of providing access to a hollow organ and facilitating the introduction of larger devices is provided. To this end, the catheter device is provided with a dilator portion arranged around the needle channel of the catheter device, comprising a bevelled distal tip, a tapered outer surface and a helix arranged around the tapered outer surface. The helix may dilate a puncture or a nick previously made in the patient's skin and the use of supplementary cutting instruments such as scalpels may be avoided. The risk of accidents manipulating cutting instruments e.g. cutting off the guide wire and/or making the puncture too deep or too long may also be avoided. The helix in combination with the bevelled distal tip facilitates entry of the dilator portion into the skin and dilation of the puncture is possible without having to exercise a lot of pressure.

Distal herein is to be understood as a side of an instrument further away from a person (e.g. nurse or doctor) using it. Proximal herein is to be understood as the opposite side.

In some examples, a distal end of the helix is located between a distal end of the bevelled distal tip and a proximal end of the bevelled distal tip. In these examples, the catheter device may be introduced with less resistance.

In some examples, a diameter of the helix increases progressively from the distal end towards the proximal end of the catheter body. The dilation can be effectively controlled by a medical professional by controlling the number of turns of the catheter device. With each turn, the catheter device may enter the skin further, and the increasing diameter will increase the dilation of the skin.

In some examples, a wire diameter of the helix increases progressively from the distal end towards the proximal end of the catheter body. The catheter device may be introduced with less resistance.

In a further aspect, a method for accessing a hollow organ is provided. The method comprises providing a catheter device according to any of the examples herein described and an elongated needle comprising a cutting end. The method further includes positioning the elongated needle in the needle channel of the catheter device such that the cutting end extends beyond the distal end of the needle channel of the catheter device. The method further includes making a puncture in a skin of a patient and the hollow organ using the cutting end of the needle while the needle is in the needle channel of the catheter device. The method further includes inserting a guide wire through the needle channel of the catheter device and dilating the puncture by screwing the dilator portion of the catheter device into the puncture.

In yet another aspect, a kit is provided. The kit includes a catheter device according to any example of the first aspect. The kit further includes a needle configured to be slidably inserted into the needle channel of the catheter device, wherein the needle comprises a cutting end which when inserted in the needle channel of the catheter device extends beyond a distal end of the catheter body. In some examples, the needle may be hollow. Potentially a guide wire could thus be passed through the needle. In some examples, a kit may further comprise a guide wire and/or a guide catheter. A surgical kit comprising all or various of the elements needed in procedures for gaining access to a lumen (e.g., a vein or urinary tract) can be provided.

Hollow organs, as used herein explicitly include bodily lumens, e.g. cavities and tubular organs. Specific examples of hollow organs include veins, and femoral or radial arteries.

### BRIEF DESCRIPTION OF THE DRAWINGS

Non-limiting examples of the present disclosure will be described in the following, with reference to the appended figures, in which:
Figure 1 schematically illustrates a sequence of situations that may occur during the performance of the Seldinger technique;
Figure 2 schematically illustrates a catheter device;
Figure 3 schematically illustrates the needle channel of the catheter device in figure 2;
Figure 4 schematically illustrates the dilator portion of the catheter device in figure 2;
Figure 5 schematically illustrates a needle device coupled with the catheter device shown in figures 2-4;
Figure 6 schematically illustrates the needle device shown in figure 5.
Figure 7 schematically illustrates the handle of the needle device shown in figures 5-6.
Figures 8a - 8g schematically illustrate a sequence of situations that may occur during the performance of a method for accessing a hollow organ according to an example.

The figures refer to example implementations and may only be used as an aid for understanding the claimed subject matter, not for limiting it in any sense.

### DETAILED DESCRIPTION OF EXAMPLES

In these figures, the same reference signs have been used to designate matching elements.

Figure 1 schematically illustrates a prior art technique for accessing a hollow organ which has hereinbefore already been discussed.

Figure 2 schematically illustrates a catheter device 100. The catheter device 100 may comprise a catheter body 104 extending longitudinally from a proximal end 104a (closer to the person handling the catheter) to a distal end 104b (the end further away from the person handling the catheter).

In some examples the catheter body 104 may be made of a polymer or mixture of polymers. The catheter body 104 may be made of any plastic suitable for any health product e.g. a PVC plastic, a cured epoxy resin, or some other suitable polymer. Such a polymer could be fibre-reinforced in some examples. The catheter body 104 may be manufactured by injection of a polymer or mixture of polymers.

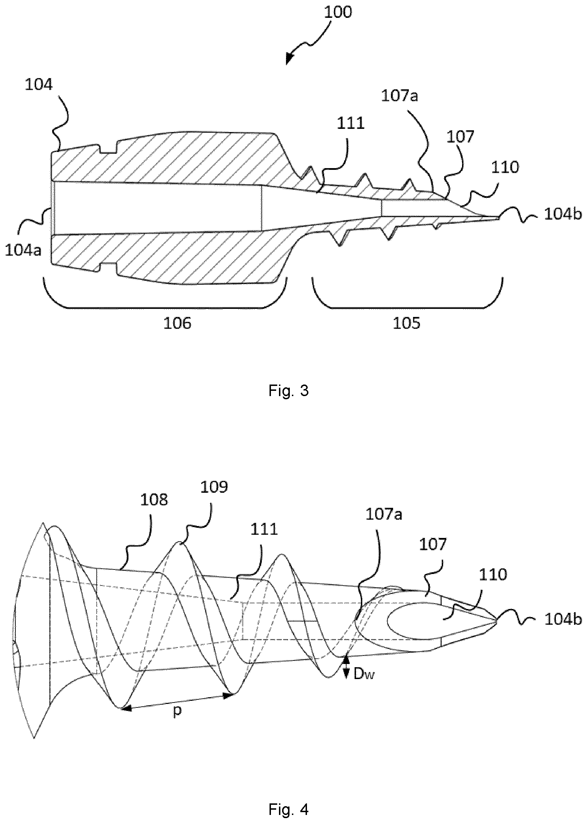

The catheter body 104 comprises a needle channel 111 arranged within the catheter body 104 and extending substantially along a longitudinal direction of the catheter body 104. A better view of the needle channel is illustrated in figure 3. The needle channel 111 comprises a distal opening 110 at the distal end 104b of the catheter body. In some examples the needle channel 111 may extend from the proximal end 104a of the catheter body to the distal end 104b of the catheter body. A through-hole is thus formed in the body.

The distal opening 110 of the needle channel 111 is formed by a bevelled distal tip 107. In some examples, the needle channel 111 may have the same diameter at the proximal end 104a of the catheter body and at the distal end 104b of the catheter body. In some other examples, the needle channel 111 may have a diameter at or near the proximal end 104a of the catheter body greater than that at or near the distal end 104b of the catheter body such that the diameter of the needle channel 111 decreases (optionally constantly) along the longitudinal length of the catheter body 104 extending from the proximal end 104a to the distal end 104b. The needle channel 111 may thus have the shape of a funnel, and the operation of, for example, a needle once inserted into the channel may be improved.

The needle channel 111 is configured to receive a removable elongated needle 212 for making a puncture in a skin when arranged in the needle channel 111. The needle channel 111 may be specifically shaped to provide a seat for a needle. The needle in question can thus be inserted in the proximal end of the device and be advanced until it encounters the seat (or "stopper"). Such a seat for a needle may be adapted to a specific needle, e.g. of a specific type or of a specific brand.

Further, coming back to figure 2, the catheter body 104 may comprise a grip portion 106 and a dilator portion 105.

The grip portion 106 may be located at or near the proximal end 104a of the catheter body 104, thus the insertion, removal and manipulation of the catheter body 104 (and thus the whole catheter) may be improved. The grip portion 106 may be shaped so that it may be easily held between the fingers of the user/surgeon. The grip portion 106 may be made of any suitable rough material e.g. plastic polymer, thus the manipulation of the catheter device 100 by the user/surgeon may be improved, especially if the grip portion 106 of the catheter is wet, or has saline or body fluids on it. The grip portion 106 may include a suitable coating, e.g. a hydrophobic coating. The grip portion 106 may comprise a textured surface, e.g. a plurality of ribs. With this arrangement, the catheter device 100 may be easily manipulated and used with great accuracy.

In some examples, the removable elongated needle 212 may be part of a needle device comprising a needle and a handle for manipulating the needle. The grip portion 106 of the catheter body 104 may further comprise at least a recess 115. In the illustrated example, an annular recess is formed in the grip portion 106 of the catheter body and extends around the circumference of the catheter body. In the illustrated example, the annular recess is formed in the proximity of the proximal end 104a.

The recess(es) 115 of the grip portion 106 of the catheter body 104 may mate with a catch on the handle of a needle device. The needle device may be coupled and uncoupled to and from the grip portion of the catheter body through the catches and the recess.

The dilator portion 105 is arranged around the needle channel 111 and is configured to enter into the puncture in the skin made by the needle and dilate the skin around the puncture. The dilator portion 105 may be configured to dilate the skin around the puncture and the underlying subcutaneous tissue. In some examples, the needle channel 111 may extend from a distal end of the dilator portion until a proximal end of the dilator portion. In figure 4, a top view of the dilator portion 105 of the catheter device of figure 2 is shown.

The dilator portion 105 comprises a bevelled distal tip 107. The bevelled distal tip 107 of the dilator portion forms the distal opening 110 of the needle channel 111. The bevelled distal tip 107 may allow an easier insertion of the catheter body 104 in a puncture made by a needle. The bevelled distal tip 107 may comprise a distal end 104b and a proximal end 107a.

In some examples, the bevelled distal tip 107 may have a length of 2 - 6 mm, specifically 3 - 5,5 mm. In examples, the distal end 104b of the bevelled distal tip 107 has a maximum thickness of 0,3 - 1 mm. The dimensions of the bevelled distal tip in these ranges have been found to facilitate entry of the dilator portion of the catheter device 100, i.e. the medical professional performing the procedure requires less skill and experience to be able to perform the procedure.

The distal opening 110 of the needle channel may have a diameter of 1 - 1.60 mm, particularly of approximately 1.30 mm. The size of the needle channel and the distal opening may be chosen such that suitably sized needles can be accommodated. In examples, the catheter device 100 may be used in combination with a needle which may have a diameter in the range e.g. 16 to 27 gauges, specifically 18 to 25 gauges depending on the expected uses of the catheter.

The dilator portion 105 further comprises a tapered outer surface 108 extending from the bevelled distal tip 107 in a proximal direction. The diameter of the tapered surface 108 may increase progressively from the distal end 104b towards the proximal end 104a of the catheter body. The dilator portion 105 further comprises a helix 109 which is arranged around the tapered outer surface 108. The helix 109 may extend rearwardly from the bevelled distal tip 107 in a proximal direction. The helix 109 may be integrally formed with the rest of the catheter body 104. The tapered outer surface 108 and the helix 109 may provide an easy and efficient way of introducing the dilator portion 105 into the skin and therefore dilating the skin.

The dilator portion 105 may be introduced in the skin through a puncture previously made by a needle 212. A medical professional may screw the catheter body 104 and introduce the helix 109 into the skin, dilating the puncture, i.e. the medical professional may rotate to the catheter body 104 while exerting some forward pressure such that the catheter body performs a substantially helical movement.

The catheter body 104 according to this example is specifically configured to dilate the puncture in the skin rather than increasing the puncture through an incision. In examples, the size of the puncture or cut is not actually increased through the action of the catheter body. Rather, the dilator portion relies on the elasticity of the skin of the subject to create an opening that is large enough to later introduce further elements such as another catheter or sheath, as will be explained herein later. In these examples, after removing the catheter body 104 (and other medical devices), the skin will revert back to its shape before elastic deformation and the puncture in the skin will substantially be the same as the original puncture of the needle.

Referring back to figure 2, the distal end 109b of the helix 109 may be located between the distal end 4b and the proximal end 107a of the bevelled distal tip 107. This may further facilitate dilating the original nick or puncture because the catheter device may be introduced with less resistance. In some examples, the distal end 109b of the helix may be closer to the proximal end 107a of the bevelled distal tip 107 than to the distal end 104b of the bevelled distal tip.

The helix 109 may comprise at least a first complete turn and a second at least partial turn. In some examples, the helix may comprise two complete turns. In examples, the helix may comprise three turns or less. The number of turns may be determined as an optimization between ease of entry into the skin, and user friendliness. A high number of turns may be uncomfortable in use, whereas a small number of turns may dilate the skin (too) rapidly as the catheter body advances.

The diameter of the helix 109 may increase progressively from the distal end 104b to the proximal end 104a of the catheter body. In some examples, a first diameter of the first turn of the helix is less than a second diameter of the second turn. In some examples, the diameter of a distal end 9b of the helix may be 1,5 - 2,5 mm and optionally a diameter of a proximal end 9a of the helix may be 4 - 5 mm. In examples, a diameter of a first distal turn of the helix may range from 1,5 - 4 mm. In some examples, the diameter of the helix may increase progressively from the distal end to the proximal end until reaching a certain value, e.g. approximately after the first complete turn and then may have a constant value towards the proximal end of the helix.

As may be seen in figure 4, the wire diameter D_{w} of the helix may progressively increase from the distal end 104b to the proximal end 104a of the catheter body. This may allow an easier introduction of the catheter body. In some examples, the wire diameter D_{w} of the helix at the distal end of the helix may be of 0,1 - 0,3 mm. In some examples the pitch p of the helix may increase progressively from the distal end to the proximal end of the catheter body.

In some examples, the dilator portion 105 of the catheter device 104 may be lubricated. Lubrication of the dilator portion may facilitate the dilating process. Any lubricant suitable for medical applications may be used. In such examples, the catheter body may be packaged with a suitable lubricant already applied to it. The medical professional may simply unpack the catheter device 100 from its packaging and use it. In other examples wherein a lubricant is used, the medical professional may apply lubricant to the device before carrying out a procedure.

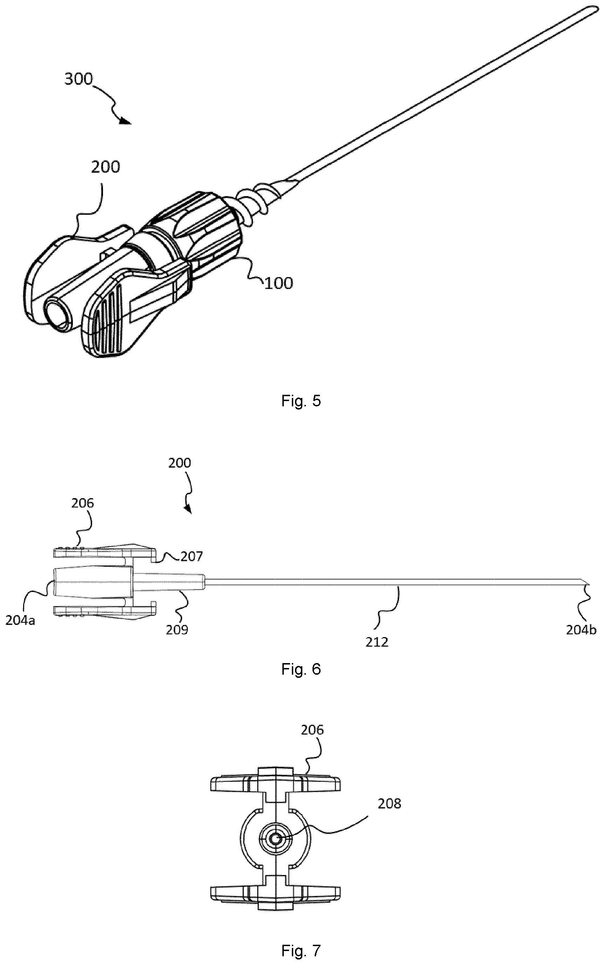

Figure 5 schematically illustrates an isometric view of an assembly 300 comprising the example of the catheter device 100 illustrated in figures 2-4 and an example of a needle device 200.

As previously explained, the catheter device 100 of this example comprises a needle channel 111 configured to receive a removable elongated needle 212. In this particular example, the removable elongated needle is part of a needle device 200 comprising the removable elongated needle 212 and a handle 206. The needle 212 may be hollow and comprise a lumen. A guidewire may be inserted through this lumen as will be explained herein after.

The needle device 200 is shown in more detail in figure 6. The needle device may extend longitudinally from a proximal end 204a to a distal end 204b and may be inserted or withdrawn along the needle channel 111 defined in the catheter body 100.

The handle 206 of the needle device 200 may have a proximal end 204a and a distal end portion 209. The needle 212 may extend from the distal end portion 209 of the handle 206. The needle may be coupled at the distal end of the handle in some examples. In other examples, the needle may extends through a part of the handle.

The needle 212 may be hollow and may include a needle lumen. The distal end 209 of the handle may have any shape which is complementary to the shape of the needle channel 111 of the catheter body 100 such that when the needle device 200 is inserted along the needle channel 111, the distal end portion 209 of the handle may be received in it. In some examples, the distal end portion 209 of the handle may have a tapered shape. In the illustrated example, the distal end portion 209 is substantially frusto-conical.

The handle 206 of the needle device 200 may comprise catches 207 which may mate with the recess 115 present in the grip portion 106 of the catheter body 104, which may allow easier manipulation of the assembly 300. As shown in figure 7, the central portion of the handle 206 may comprise an opening 208 connecting with a handle lumen. In this example, the handle lumen may be aligned with and continue in the lumen of the needle. A wire inserted into a proximal end 204a of the handle may thus be advanced into the lumen of the needle.

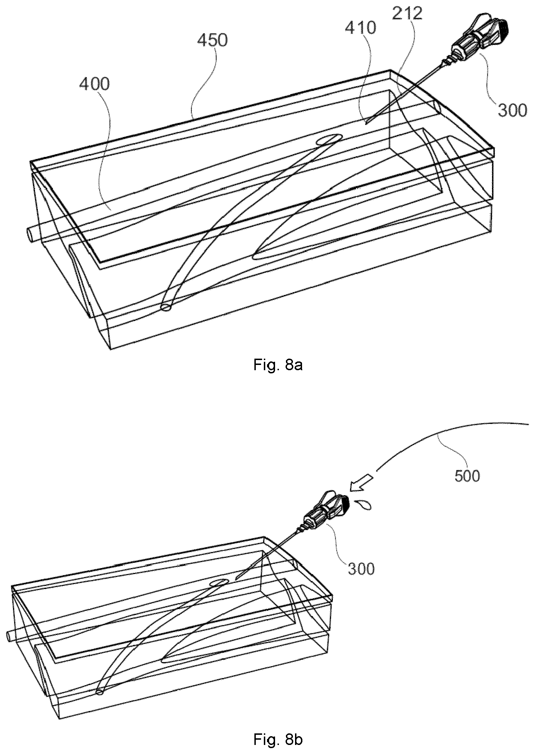

In figure 8a, a hollow organ 400 e.g. a blood vessel, may be seen that is located under a patient's skin. In figure 8a, an example of a catheter device 100 similar to the previous examples is shown.

The catheter device 100 comprises a dilator portion 105 arranged around a needle channel 111. The dilator portion 105 comprises a bevelled distal tip 107, a tapered outer surface 108 and a helix 109 arranged around the tapered outer surface 108.

The removable elongated needle 212 may comprise a cutting end and at the opposite end a handle or grip can be foreseen. The needle 212 may thus be inserted or withdrawn along the needle channel 111 by the through-hole defined in the catheter body 104.

The removable elongated needle 212 may be a hollow needle comprising a lumen configured to allow the insertion and removal of a guide wire 500 through the lumen (and thus through the needle). The removable elongated needle may have a diameter in the range e.g. 16 to 27 gauges, specifically 18 to 25 gauges depending on the expected uses of the catheter.

The removable elongated needle could be pre-assembled with the catheter device, thus forming a pre-assembled kit. Alternatively, the needle and the catheter device can be delivered separately as a set of parts, in which case a surgeon or nurse assembles the removable elongated needle on the catheter device in preparation for use.

An assembly 300 comprising a needle device and a catheter device may be provided. Figure 8a illustrates an initial situation. The removable hollow elongated needle 212 is arranged in the needle channel 111 of the catheter device 104, which is configured to receive a removable elongated needle. The cutting end of the elongated needle 212 extends beyond the distal end 104b of the catheter body 104 (and thus the distal end of the bevelled distal tip). This way, the cutting end is ready to make a puncture in a patient's skin and enter into the vein. In some examples, the grip portion 106 of the catheter body 104 comprises a recess 115. In this particular example, the needle 212 inserted in the needle channel 111 is part of a needle device 200, comprising a handle 206 and the needle 212. The handle 206 of the needle device may comprise at least a catch 207 suitable for mating with at least a recess 115 in the catheter device 104.

In figure 8a, the cutting end of the removable elongated needle 212 may be brought near the patient's skin 450. Once situated at the desired position, the needle 212 may be used to make a puncture 410 in the skin. Once the removable elongated needle reaches the vein 400, blood may drip out at a proximal end of the catheter (e.g. the proximal end of a hollow needle), thus indicating the user/surgeon that the needle has been properly placed into the hollow organ.

In figure 8b, a guide wire 500 configured to be slidably inserted and removed through a passage or a lumen may be provided. In this example, the guide wire 500 may be inserted into the hollow needle 212 and therefore through the needle channel 111 of the catheter body 104. The guide wire 500 may have a suitable diameter in order to be inserted into the needle in the direction pointed by the arrow. The guide wire 500 may further have a low coefficient of friction, thus the insertion and removal may be improved. The guide wire 500 may be made e.g. of stainless steel or nitinol, but other materials are possible as well.

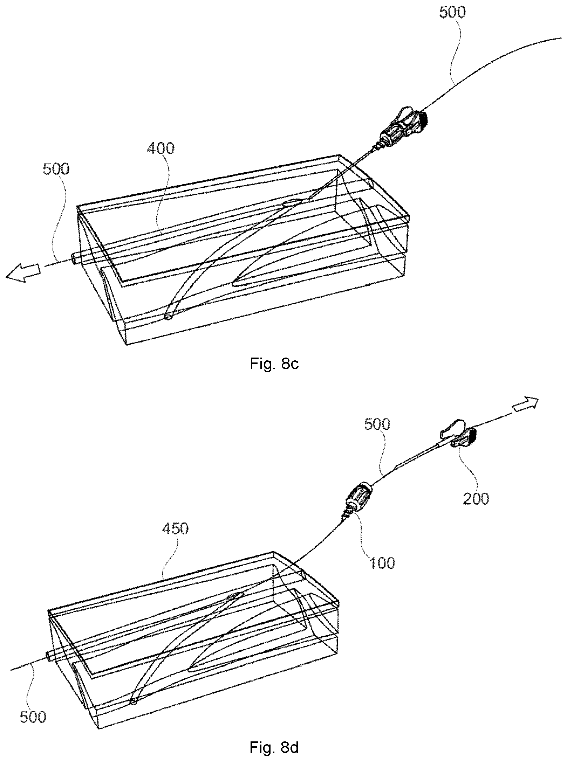

In figure 8c, the distal end of the guide wire may be inserted into the hollow needle. The user/surgeon may hold and manually manoeuvre the proximal end of the guide wire 500, thus the guide wire may be properly positioned in the hollow organ, i.e. herein the vein. In some examples, the guide wire may have a handle located at or near the proximal end of the guide wire 500, thus facilitating the control of the guide wire.

After the guide wire 500 has been properly positioned in the hollow organ, the hollow needle may be withdrawn, see figure 8d. The needle device 200 may be uncoupled from the catheter device 100 by releasing the catches 207 in the handle of the needle device from the annular recess 115 in the catheter body. After withdrawing the hollow needle, the guide wire 500 may remain in the hollow organ and inside the needle channel 111 of the catheter device 100, extending beyond the bevelled distal tip 107 of the catheter body 104.

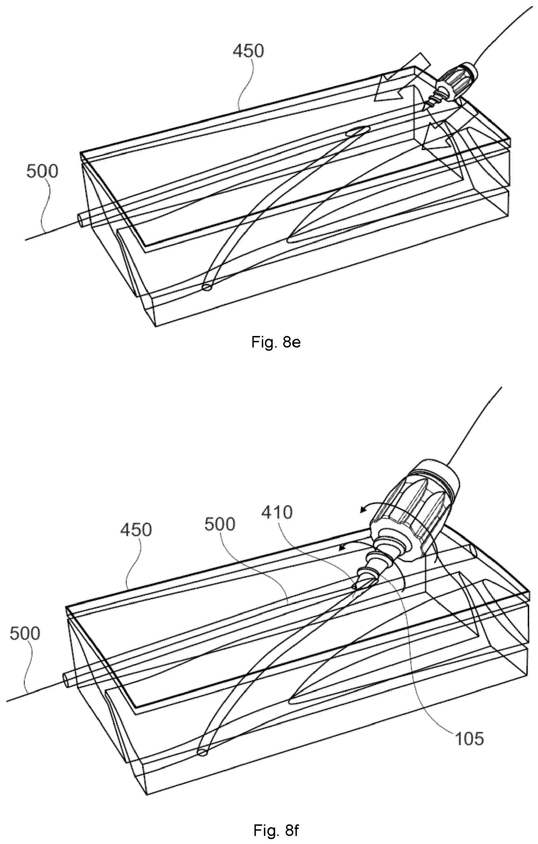

In figure 8e the catheter body 104 may be advanced over the guide wire 500 and the bevelled distal tip 107 of the catheter device may then be entered into the puncture 410 in the skin made by the removable elongated needle. In some examples the distal part 109b of the helix 109 may be located between the distal and proximal ends 104b, 107a of the bevelled tip and may enter into the puncture with the bevelled distal tip. Thereafter, as shown in figure 8f, the user may lightly push the catheter body 104 forwards while rotating it. The catheter body 104 may thus perform a helical or "screwing" movement.

The dilator portion 105 of the catheter body 104 may enter into the puncture 410 in the skin made by the removable elongated needle 212. As a result, the puncture 410 may be dilated. Since the guide wire may remain in the needle channel of the catheter device and dilating is achieved by turning/screwing the catheter body, dilation of the skin may be achieved without altering the integrity of the guide wire.

In some examples, turning/screwing the catheter body comprises entering at least two turns of the helix into the skin. With each turn of the catheter body, the puncture in the skin may be dilated more. In some examples, the puncture may be dilated with two turns of the catheter body. In some examples the puncture may be dilated between 3-4 mm.

In some examples, the distal end of the catheter device may be lubricated. Lubrication of the distal end may facilitate insertion of the catheter device into the skin.

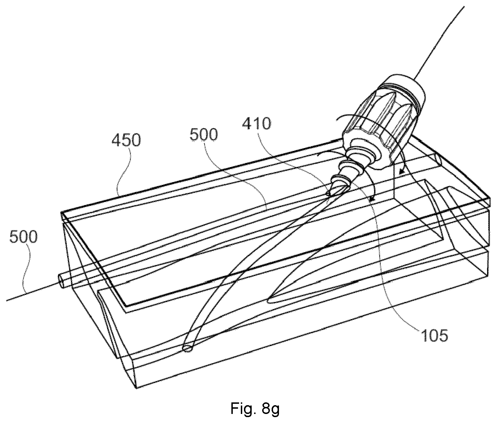

Once the puncture has been enlarged, the catheter body 104 may be withdrawn by unscrewing the catheter device 100, as illustrated in figure 8g. The guide wire 500 may still remain in the hollow organ. A treatment catheter or sheath, or any other medical instrument that may be used in a subsequent procedure and which may be larger than the guide wire, may then be inserted into the hollow organ through the dilated puncture and the guide wire.

A treatment catheter or a sheath may comprise a conical tapered distal portion that is narrower compared to the proximal portion of the catheter. The treatment catheter may thus act as a dilator to facilitate advancement of the treatment catheter through the hollow organ. Moreover, the treatment catheter may provide additional stiffness or reinforcement in the wall of the hollow organ. Fluoroscopy may be used to confirm the position of the catheter and to manoeuvre it to the desired location. Depending on the intervention, once the treatment catheter has reached its desired position, the guide wire may be removed.

In examples, a treatment catheter may be directly positioned inside the vein or other cavity. In some examples, a sheath may first be positioned which functions as a passageway for catheter(s) to be used afterwards.

After completion of an intervention, the treatment catheter may be withdrawn. In some examples, a sealing device may be used to close the incision made by the procedure.

Although not illustrated in any of the examples in the figures, the catheter body 104 may comprise an insertion tube arranged at a distal end of the catheter body (at a distal end of the dilator portion) 104b, the insertion tube comprising a tubular elongate passage aligned with the needle channel of the catheter body to define a lumen. In some examples, the insertion tube may be made of a flexible material. The insertion tube may facilitate the introduction of the tube into the hollow organ and may reduce the chances of the hollow organ being punctured or damaged by e.g. sudden or accidental movements. The insertion tube may be made of a suitable plastic, particularly PTFE. The procedure for accessing e.g. a vein through the patient's skin may be improved hereby.

The catheter may be held and used in the same way as traditional trocars or Abbocaths ("hollow needle within a tube", typically made of Teflon). In a very similar manner as in the traditional method, the initial puncture may be made by the needle. In examples wherein a catheter device 100 including an insertion tube is used, the needle 212, which may or may not be hollow, can then be immediately removed after making the puncture. A guide wire may then be introduced into the organ (vein) through the needle channel 111 in the catheter device that previously surrounded the needle and through the insertion tube. Once in place, the catheter body 104 just needs to be pushed forward while it is being rotated along the guide wire performing a helical or "screwing" movement in order to dilate the skin in a very controlled manner. In this particular example, since the needle stays in place less time, the risk of an accidental movement by a nurse or other healthcare professional potentially cutting skin or another organ is reduced.

For reasons of completeness, various aspects of the present disclosure are set out in the following clauses:
Clause 1. A catheter device (100) configured to provide access to a hollow organ comprising:
   a catheter body (104) extending longitudinally from a proximal end (104a) to a distal end (104b), wherein the catheter body (104) comprises:
   a needle channel (111) arranged within the catheter body (104) and extending substantially along a longitudinal direction of the catheter body (104) and comprising a distal opening (110) at the distal end (104b) of the catheter body (104), wherein the needle channel (111) is configured to receive a removable elongated needle (112) for making a puncture in a skin when arranged in the needle channel (111); and further comprises
   a dilator portion (105) arranged around the needle channel (111) and configured to enter into the puncture in the skin made by the removable elongated needle (112) and configured to dilate the skin around the puncture, wherein
   the distal opening (110) of the needle channel (111) is formed by a bevelled distal tip (107) of the dilator portion (105), and the dilator portion (105) further comprises
   a tapered outer surface (108) extending from the bevelled distal tip (107) in a proximal direction; and
   a helix (109) arranged around the tapered outer surface (108).
Clause 2. The catheter device (100) according to clause 1, wherein a distal end (109a) of the helix (109) is located between a distal end (104b) of the bevelled distal tip and a proximal end (107a) of the bevelled distal tip.
Clause 3. The catheter device (100) according to clause 2, wherein the distal end (109b) of the helix (109) is closer to the proximal end (107a) of the bevelled distal tip than to the distal end (104b) of the bevelled distal tip.
Clause 4. The catheter device (100) according to any of clauses 1-3, wherein a length of the bevelled distal tip (107) is 2 - 6 mm, specifically 3 - 5,5 mm.
Clause 5. The catheter device (100) according to clauses 1-4, wherein the distal end (104b) of the bevelled distal tip (107) has a maximum thickness of 0,3 - 1 mm.
Clause 6. The catheter device (100) according to any of clauses 1-5, wherein the helix (109) comprises at least a first complete turn and a second at least partial turn, optionally wherein the second turn is a complete turn.
Clause 7. The catheter device (100) according to clause 6, wherein a first diameter of the first turn of the helix (109) is less than a second diameter of the second turn of the helix (109).
Clause 8. The catheter device (100) according to any of clauses 1-7, wherein a diameter of the helix (109) increases progressively from the distal end (104b) towards the proximal end (104a) of the catheter body.
Clause 9. A catheter device (100) according to any of clauses 1-8, wherein the diameter of a distal end (9b) of the helix is 1,5- 2,5 mm and optionally a diameter of a proximal end (9a) of the helix is 4 - 5 mm.
Clause 10. A catheter according to any of clauses 6-9, wherein a diameter of a first distal turn of the helix is of 1,5 - 4 mm.
Clause 11. The catheter device (100) according to any of clauses 1 - 10, wherein a wire diameter (D_{w}) of the helix (109) increases progressively from the distal end (104b) to the proximal end (104a) of the catheter body.
Clause 12. The catheter device (100) according to any of clauses 1 - 11, wherein a wire diameter (D_{w}) of the helix at the distal end (109b) of the helix is of 0,1 - 0,3 mm.
Clause 13. The catheter device (100) according to any of clauses 1 - 12, wherein a pitch (p) of the helix increases progressively from the distal end (104b) to the proximal end (104a) of the catheter body.
Clause 14. The catheter device (100) according to any of clauses 1 - 13, further comprising a grip portion (106) located at or near the proximal end (104a) of the catheter body and configured to manipulate the catheter body (104).
Clause 15. The catheter device (100) according to any of clauses 1 - 14, wherein the grip portion (106) comprises one or more recesses (115) adapted to mate with one or more catches of a handle of a needle device, wherein the needle device comprises a removable elongated needle (112) and a handle.
Clause 16. The catheter device (100) according to any of clauses 1 - 15, wherein the dilator portion (105) of the catheter body (104) is lubricated.
Clause 17. The catheter device (100) according to any of clauses 1 - 16, wherein the catheter body (104) is made of a polymer or mixture of polymers.
Clause 18. The catheter device (100) according to any of clauses 1 - 17, further comprising an insertion tube arranged at the distal end of the catheter body.
Clause 19. A method for accessing a hollow organ comprising:
   providing a catheter device according to any of clauses 1-18;
   providing an elongated needle comprising a cutting end;
   positioning the elongated needle in the needle channel of the catheter device such that the cutting end extends beyond the distal end of the needle channel of the catheter device;
   making a puncture in a skin of a patient and the hollow organ using the cutting end of the needle while in the needle channel of the catheter device;
   inserting a guide wire through the needle channel and into the hollow organ;
   dilating the puncture by screwing the dilator portion of the catheter device into the puncture.
Clause 20. The method according to clause 19, wherein dilating the puncture is achieved in at least one turn of the catheter device.
Clause 21. The method according to any of clauses 19 - 20, wherein dilating the puncture includes dilating the puncture to a size of at least 3-4 mm.
Clause 22. The method according to any of clauses 19 - 21, further comprising removing the catheter device over the guide wire.
Clause 23. The method according to clause 22, wherein removing the catheter device includes unscrewing the catheter device.
Clause 24. The method according to any of clauses 17 - 23, wherein the catheter device comprises an insertion tube at a distal end of the catheter body.
Clause 25. The method according to clause 24, wherein the needle is removed before inserting the guide wire.
Clause 26. The method according to any of clauses 17 - 24, wherein the needle is hollow, and inserting the guide wire comprises inserting the guide through the hollow needle while the needle is positioned at least partially in the needle channel.
Clause 27. The method according to clause 26, further comprising withdrawing the needle after inserting the guide wire.
Clause 28. The method according to any of clauses 19 - 27, wherein the needle is withdrawn before dilating the puncture.
Clause 29. The method according to any of clauses 19 - 28, further comprising passing a treatment catheter over the guide wire.
Clause 30. A kit including:
   the catheter device according to any of clauses 1-18;
   an elongated needle device configured to be slidably inserted into the needle channel of the catheter device, wherein
   the needle device comprises a handle and a needle, wherein the needle comprises a cutting end which when inserted in the needle channel of the catheter device extends beyond a distal end of the needle channel.
Clause 31. The kit according to clause 30, wherein the handle of the needle device comprises at least a catch configured to couple to at least a recess on the grip portion of the catheter device.
Clause 32. The kit according to any of clauses 30 - 31, wherein the elongated needle is a hollow needle comprising a channel.

Although only a number of examples have been disclosed herein, other alternatives, modifications, uses and/or equivalents thereof are possible. Furthermore, all possible combinations of the described examples are also covered. Thus, the scope of the present disclosure should not be limited by particular examples, but should be determined only by a fair reading of the claims that follow.

## Claims

1. A catheter device (100) configured to provide access to a hollow organ comprising:
a catheter body (104) extending longitudinally from a proximal end (104a) to a distal end (104b), wherein the catheter body (104) comprises:
a needle channel (111) arranged within the catheter body (104) and extending substantially along a longitudinal direction of the catheter body (104) and comprising a distal opening (110) at the distal end (104b) of the catheter body (104), wherein the needle channel (111) is configured to receive a removable elongated needle (112) for making a puncture in a skin when arranged in the needle channel (111); and further comprises
a dilator portion (105) arranged around the needle channel (111) and configured to enter into the puncture in the skin made by the removable elongated needle (112) and configured to dilate the skin around the puncture, wherein
the distal opening (110) of the needle channel (111) is formed by a bevelled distal tip (107) of the dilator portion (105), and the dilator portion (105) further comprises
a tapered outer surface (108) extending from the bevelled distal tip (107) in a proximal direction; and
a helix (109) arranged around the tapered outer surface (108).

2. The catheter device (100) according to claim 1, wherein a distal end (109a) of the helix (109) is located between a distal end (104b) of the bevelled distal tip and a proximal end (107a) of the bevelled distal tip.

3. The catheter device (100) according to claim 2, wherein the distal end (109b) of the helix (109) is closer to the proximal end (107a) of the bevelled distal tip than to the distal end (104b) of the bevelled distal tip.

4. The catheter device (100) according to any of claims 1-3, wherein a length of the bevelled distal tip (107) is 2 - 6 mm, specifically 3 - 5,5 mm.

5. The catheter device (100) according to any of claims 1-4, wherein the helix (109) comprises at least a first complete turn and a second at least partial turn, optionally wherein the second turn is a complete turn.

6. The catheter device (100) according to any of claims 1-5, wherein a diameter of the helix (109) increases progressively from the distal end (104b) towards the proximal end (104a) of the catheter body.

7. The catheter device (100) according to any of claims 1-6, wherein a wire diameter (D_{w}) of the helix (109) increases progressively from the distal end (104b) to the proximal end (104a) of the catheter body.

8. The catheter device (100) according to any of claims 1-7, wherein a pitch (p) of the helix increases progressively from the distal end (104b) to the proximal end (104a) of the catheter body.

9. The catheter device (100) according to any of claims 1-8, further comprising a grip portion (106) located at or near the proximal end (104a) of the catheter body and configured to manipulate the catheter body (104).

10. The catheter device (100) according to any of claims 1-9, wherein the grip portion (106) comprises one or more recesses (115) adapted to mate with one or more catches of a handle of a needle device, wherein the needle device comprises a removable elongated needle (112) and a handle.

11. The catheter device (100) according to any of claims 1 - 10, wherein the dilator portion (105) of the catheter body (104) is lubricated.

12. The catheter device (100) according to any of claims 1 - 11, further comprising an insertion tube arranged at the distal end of the catheter body.

13. A kit including:
the catheter device according to any of claims 1 - 12;
an elongated needle device configured to be slidably inserted into the needle channel of the catheter device, wherein
the needle device comprises a handle and a needle, wherein the needle comprises a cutting end which when inserted in the needle channel of the catheter device extends beyond a distal end of the needle channel.

14. The kit according to claim 13, wherein the handle of the needle device comprises at least a catch configured to couple to at least a recess on the grip portion of the catheter device.

15. The kit according to any of claims 13 - 14, wherein the elongated needle is a hollow needle comprising a channel.
